# EUROPÄISCHE PATENTSCHRIFT

(11) **EP 1 093 763 B1**
(45) Veröffentlichungstag und Bekanntmachung des Hinweises auf die Patenterteilung: **07.03.2007**
(21) Anmeldenummer: 00113452.7
(22) Anmeldetag: 24.06.2000
(51) Int. Cl.: A61B 17/80, A61B 17/70

(54) **Knochenplatte**
Bone plate
Plaque d'ostéosynthèse

(30) Priorität: 18.10.1999 DE 19950252
(43) Veröffentlichungstag der Anmeldung: 25.04.2001
(73) Patentinhaber: DePuy Spine Sàrl, 2400 Le Locle (CH)
(72) Erfinder: Schäfer, Bernd, 6315 Oberägeri (CH); Halm, Henry, Dr., 49143 Bissendorf (DE); Liljenqvist, Ulf, Dr., 48147 Münster (DE); Chan, Donald, Prof. Dr., Charlottesville VA 22901 (US)
(74) Vertreter: Dreiss, Fuhlendorf, Steimle & Becker

(56) Entgegenhaltungen:
- DE-A- 4 414 782
- DE-A- 4 433 360
- DE-A- 19 509 331

## Beschreibung

Die Erfindung betrifft eine Knochenplatte für die Osteosynthese mit den Merkmalen des Oberbegriffs des Anspruchs 1.

Knochenplatten für die Osteosynthese sind hinreichend bekannt. Derartige Knochenplatten werden z.B. an Wirbeln befestigt, um die Wirbel zu stabilisieren. Hierfür werden die einzelnen Knochenplatten über Stäbe miteinander verbunden, wobei die Stäbe an den Knochenplatten befestigt, insbesondere festgeklemmt werden. Zum Befestigen der Knochenplatten an den Wirbeln werden Knochenschrauben verwendet, die die Knochenplatten durchdringen und in den Wirbel eingeschraubt werden. Die Knochenplatte wird in der Regel mittels des Schraubenkopfes festgehalten. Aus der DE-A-44 33 360 ist eine Haltevorrichtung bekannt geworden, die mit zwei Knochenschrauben am Knochen befestigt wird. Der Fixierstab wird mit einem Deckel am Grundkörper festgeklemmt.

Es hat sich gezeigt, dass insbesondere bei der thorakalen Anwendung Knochenplatten mit geringer Baulänge zu bevorzugen sind. Außerdem sollen die Knochenplatten eine geringe Bauhöhe aufweisen. Diese Anforderungen werden jedoch nicht von Knochenplatten erfüllt, welche mittels mehrerer Knochenschrauben am Knochen, insbesondere am Wirbel befestigt werden.

Die DE-A-44 14 782 zeigt eine Haltevorrichtung, die ebenfalls mit zwei Schrauben am Knochen befestigt wird. Der Fixierstab wird aber in einen am Grundkörper vorgesehenen Gabelkopf eingelegt und mittels einer Mutter befestigt.

Aus der DE-A-195 09 331 ist eine Vorrichtung zum Befestigen eines Fixierstabes an einem Knochen bekannt, die die Merkmale des Oberbegriffs des Anspruchs 1 aufweist. Dabei wird der Grundkörper mit einer einzigen Knochenschraube am Knochen fixiert. Dieser Grundkörper weist neben der Aufnahmeöffnung für den Kopf der Knochenschraube einen gabelkopfförmigen Aufnahmebereich mit einem Außengewinde auf, in welchen der Fixierstab eingelegt und mittels einer Mutter befestigt wird. Diese Vorrichtung besitzt eine große Bauhöhe.

Der Erfindung liegt daher die Aufgabe zugrunde, eine Knochenplatte bereitzustellen, welche problemlos thorakal eingesetzt werden kann.

Diese Aufgabe wird mit einer Knochenplatte gelöst, die die Merkmale des Anspruchs 1 aufweist.

Die erfindungsgemäße Knochenplatte wird also wie üblich in den Knochen eingeschlagen und mittels einer Knochenschraube befestigt. Für die Befestigung wird jedoch lediglich eine einzige Knochenschraube verwendet, die in die hierfür vorgesehene einzige Aufnahmeöffnung eingeführt wird. Die Knochenschraube hält über diese Aufnahmeöffnung die Knochenplatte am Knochen fest. Da lediglich eine einzige Knochenschraube verwendet wird, bedarf es lediglich einer Aufnahmeöffnung, so dass die Baulänge der Knochenplatte erheblich reduziert wird. Der thorakale Einsatz dieser Knochenplatte bereitet daher keinerlei Probleme. Dabei ist erfindungsgemäß der Grundkörper mit einem Aufnahmebereich für den Fixierstab versehen und ist die Aufnahmeöffnung innerhalb dieses Aufnahmebereichs vorgesehen. Somit entfallen zusätzliche Bereiche, in welchen die Aufnahmeöffnung für die Knochenschraube anzuordnen ist. Da der Aufnahmebereich für den Fixierstab ohnehin im Grundkörper vorgesehen ist, kann in diesem auch die Aufnahmeöffnung für den Schraubenkopf der Knochenschraube platziert sein.

Insbesondere befindet sich die Aufnahmeöffnung unterhalb des Fixierstabes. Der Fixierstab erstreckt sich also oberhalb des Schraubenkopfes der Knochenschraube. Dies erlaubt eine optimale Krafteinleitung der vom Fixierstab ausgeübten Kräfte in den Knochen, ohne dass die Knochenplatte mit großen Momenten beaufschlagt wird, die ebenfalls abgestützt werden müssten.

Hierzu trägt auch der Umstand bei, dass die Aufnahmeöffnung symmetrisch innerhalb des Aufnahmebereichs vorgesehen ist. Die Aufnahmeöffnung liegt bevorzugt auf der Mittelsymmetrielinie bzw. in der vertikalen Mittelsymmetrieebene der Knochenplatte.
Mit Vorzug ist die Aufnahmeöffnung als Senkaufnahme für einen als Senkkopf ausgebildeten Schraubenkopf der Knochenschraube ausgebildet. Dabei ist die Aufnahmeöffnung bevorzugt kalottenförmig ausgeführt, und der Schraubenkopf der Knochenschraube ballig ausgebildet. Auf diese Weise wird zum einen der Grundkörper der Knochenplatten optimal gehalten zum anderen bedarf es für die Aufnahme des Schraubenkopfes nur einer geringen Bauhöhe.

Bei einer Ausführungsform ist die Aufnahmeöffnung zumindest über einen Teilbereich ihrer dem Schraubenkopf zugewandten Fläche mit einer Oberflächenstruktur versehen.

Aufgrund der Oberflächenstruktur in der Aufnahmeöffnung, an welcher der Schraubenkopf anliegt, wird eine Rückhaltewirkung, d.h. Ausdrehrichtung für den Schraubenkopf erzielt. Die Knochenplatte wird dadurch nicht nur kraftschlüssig sondern auch formschlüssig mit dem Schraubenkopf und somit mit der Schraube verbunden. Aufgrund des Formschlusses wird die Gefahr verringert, dass sich die Schraube lockert, d.h. den festen Halt im Knochen verliert. Außerdem besteht nach wie vor ein Verbund zwischen Schraubenkopf und Knochenplatte, auch wenn der Knochen im Bereich der Anlage an der Knochenplatte seine Form verändert.

Bevorzugt ist die Aufnahmeöffnung kreisrund ausgebildet. Derartige Öffnungen erlauben ein problemloses Einschrauben und Anliegen des Schraubenkopfes.

Obwohl die Oberflächenstruktur lediglich über einen Teilbereich des Umfanges der Aufnahmeöffnung vorgesehen sein muß, erstreckt sich die Oberflächenstruktur bei einem bevorzugten Ausführungsbeispiel über den gesamten Innenumfang der Aufnahmeöffnung. Dies hat den wesentlichen Vorteil, dass der Kopf der Knochenschraube ebenfalls über seinen gesamten Außenumfang fixiert wird, indem er formschlüssig in der Aufnahmeöffnung verankert ist.

Eine Weiterbildung sieht vor, dass der vom Knochen abgewandte Bereich der Aufnahmeöffnung eine Oberflächenstruktur aufweist. Insbesondere bei kalottenförmig ausgebildeten Aufnahmeöffnungen, in welche ein ballig ausgebildeter Kopf der Knochenschraube eingesetzt wird, ist der nahezu senkrecht verlaufende, d.h. weniger geneigte, vom Knochen abgewandte Bereich der Aufnahmeöffnung mit der Oberflächenstruktur versehen, wodurch der Halt des Schraubenkopfes sicherer ist als im geneigten Bereich. In dem Bereich, welcher im wesentlichen senkrecht zur Achse der Schraube ausgerichtet ist, bewegt sich der Schraubenkopf beim Einschrauben der Schraube im wesentlichen parallel zur und entlang der Innenoberfläche der Aufnahmeöffnung. Erst unmittelbar am Ende des Einschraubvorganges legt sich der Schraubenkopf mit seinem unteren Bereich auf den geneigten Abschnitt der kalottenförmig ausgeführten Aufnahmeöffnung auf und hält dadurch die Knochenplatte am Knochen fest.

Die folgenden Ausführungsformen gehören nicht zur beanspruchten Erfindung:
Bevorzugt weist die Oberflächenstruktur einen in Umfangsrichtung gerichteten Strukturverlauf auf. Auf diese Weise wird eine Hemmung in Umfangsrichtung, d.h. in Drehrichtung der Schraube erzielt.

Bevorzugte Ausführungsformen sehen vor, dass die Oberflächenstruktur in Form von Längsnuten, einer Verzahnung, einer Riffelung oder dgl. ausgebildet ist. Es ist auch denkbar, dass die Oberflächenstruktur durch eine Aufrauhung der Oberfläche geschaffen wird.

Eine bevorzugte Ausführungsform wird darin gesehen, dass die Längsnuten oder die Verzahnung in Form einer Sägeverzahnung ausgebildet ist. Dabei weist jeder Sägezahn der Sägeverzahnung eine steile und eine flache Flanke auf. Um eine Hemmwirkung in Aufschraubrichtung der Schraube zu erzielen, steigt die flache Flanke in Einschraubrichtung der Knochenschraube an. Die Knochenschraube kann daher relativ leicht eingeschraubt werden und wird von der steilen Flanke des Sägezahns der Sägeverzahnung gegen ein Ausschrauben gesichert.

Eine Optimierung der Hemmwirkung wird dadurch erzielt, dass der Schraubekopf mit einer Oberflächenstrukturierung versehen ist, die die Hemmwirkung unterstützt. Insbesondere kann der Schraubenkopf ebenfalls mit in Längsrichtung verlaufenden Nuten oder dgl. versehen sein. Auch eine Sägeverzahnung ist denkbar.

Weitere Vorteile, Merkmale und Einzelheiten der Erfindung ergeben sich aus der nachfolgenden Beschreibung, in der unter Bezugnahme auf die Zeichnung ein besonders bevorzugtes Ausführungsbeispiel im Einzelnen beschrieben ist.
- Figur 1: eine Seitenansicht einer Knochenplatte mit in Schließstellung sich befindenden Deckel;
- Figur 2: eine perspektivische Ansicht eines Grundkörpers der Knochenplatte mit abgenommenem Deckel; und
- Figur 3: eine Draufsicht auf den Grundkörper bei geöffnetem Deckel.

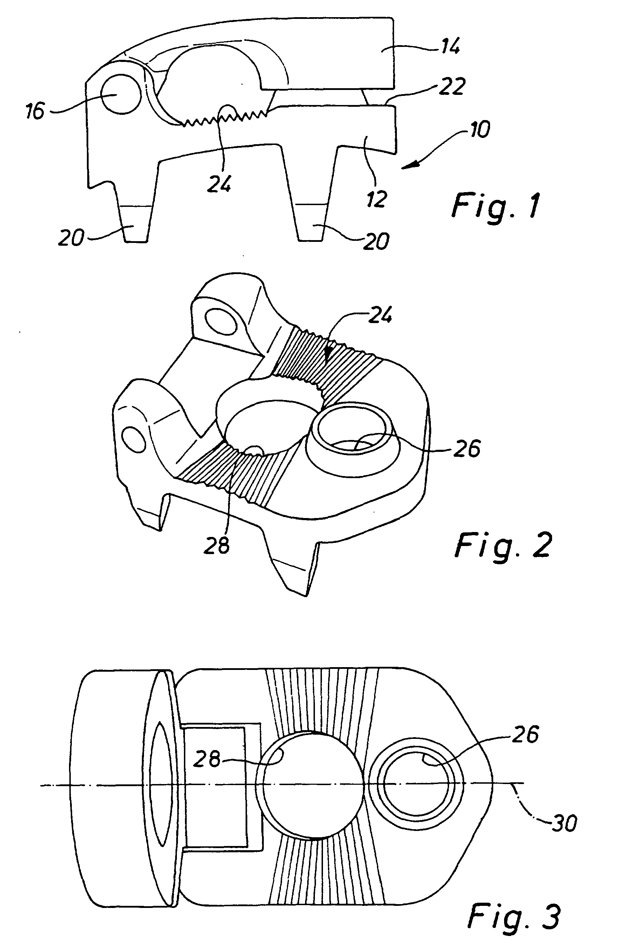

Die Figur 1 zeigt eine insgesamt mit 10 bezeichnete Knochenplatte, welche einen Grundkörper 12 und einen Deckel 14 aufweist. Der Deckel 14 ist über ein Scharnier 16 am Grundkörper 12 verschwenkbar befestigt. Der Grundkörper 12 weist an seiner, dem Knochen zugewandten Unterseite 18 insgesamt vier Ankerkeile 20 auf (von denen lediglich zwei sichtbar sind). Diese Ankerkeile 20 werden in den Knochen eingeschlagen, bis die Unterseite 18 auf der Knochenoberfläche aufliegt. Die Oberseite 22 des Grundkörpers 12 ist mit einem Aufnahmebereich 24 für einen Fixierstab versehen, welcher in diesen Aufnahmebereich eingelegt wird. Die Befestigung des Fixierstabes auf dem Grundkörper 12 im Aufnahmebereich 24 erfolgt durch das Aufschwenken des Deckels 14, welcher mittels einer Schraube, die über eine Gewindebohrung 26 (Figuren 2 und 3) mit dem Grundkörper 12 verschraubt wird.

In den Figuren 2 und 3 ist eine Aufnahmeöffnung 28 erkennbar, die einen Schraubenkopf einer Knochenschraube aufnimmt. Mittels dieser Knochenschraube wird die Knochenplatte 10 am Knochen fixiert. Die Aufnahmeöffnung 28 ist kalottenförmig ausgeführt und liegt in der Längsmittelebene 30 der Knochenplatte 10. Außerdem befindet sich die Aufnahmeöffnung 28 im Aufnahmebereich 24 für den Fixierstab. Nach dem Einsetzen der Knochenschraube in die Aufnahmeöffnung 28 und Befestigen der Knochenplatte 10 am Knochen mittels der Knochenschraube nimmt die Aufnahmeöffnung 28 den Schraubenkopf vollständig auf, so dass keine Kollision zwischen der Knochenschraube und dem Fixierstab entsteht, d.h. der Fixierstab problemlos in den Aufnahmebereich 24 eingelegt und vom Deckel 14 festgehalten werden kann. Es ist auch denkbar, dass die Aufnahmeöffnung 28 einen Versatz zur Längsmittelebene 30 aufweist. Dies ist insbesondere dann von Vorteil, wenn Knochenschrauben mit einem Schraubenkopf mit größerem Durchmesser verwendet werden. Dann können Aufnahmeöffnungen 28 mit größerem Durchmesser vorgesehen sein. Andererseits kann durch den Versatz der Aufnahmeöffnung 28 zur Längsmittelebene 30 die Länge der Platte aber auch geringfügig verkürzt werden.

## Patentansprüche

1. Knochenplatte (10) für die Osteosynthese mit einem plattenförmigen Grundkörper (12), wobei der Grundkörper (12) eine einzige Aufnahmeöffnung (28) zur Aufnahme einer den Grundkörper (12) z.B. an einem Wirbel befestigenden Knochenschraube aufweist, indem der Schraubenkopf der Knochenschraube von der Aufnahmeöffnung (28) aufgenommen wird, wobei der Grundkörper (12) mit einem Aufnahmebereich (24) für einen Fixierstab versehen ist, wobei die Befestigung des Fixierstabes auf dem Grundkörper (12) im Aufnahmebereich (24) durch einen Deckel (14) erfolgt, **dadurch gekennzeichnet, dass** der Deckel (14) über ein Scharnier (16) schwenkbar am Grundkörper (12) befestigt ist und dass die Aufnahmeöffnung (28) innerhalb dieses Aufnahmebereichs (24) vorgesehen ist.

2. Knochenplatte nach Anspruch 1, **dadurch gekennzeichnet, dass** die Aufnahmeöffnung (28) symmetrisch innerhalb des Aufnahmebereichs (24) vorgesehen ist.

3. Knochenplatte nach einem der vorhergehenden Ansprüche, **dadurch gekennzeichnet, dass** die Aufnahmeöffnung (28) als Senkaufnahme für einen als Senkkopf ausgebildeten Schraubenkopf der Knochenschraube ausgebildet ist.

4. Knochenplatte nach einem der vorhergehenden Ansprüche, **dadurch gekennzeichnet, dass** die Aufnahmeöffnung (28) kalottenförmig ausgebildet ist.

## Claims

1. A bone plate (10) for osteosynthesis comprising a plate-shaped base (12), with the base (12) having a single bone-screw receiving opening (28) for receiving a bone screw that attaches it to a bone, with the screw head of the bone screw being received by the bone-screw receiving opening (28), wherein the base (12) has a setting-rod receiving area (24) for a setting-rod and wherein the fixation of the setting-rod onto the base (12) in the setting-rod receiving area (24) is achieved by a top (14), **characterized in that** the top (14) is pivotally attached to the base (12) by a hinge (16) and that the bone-screw receiving opening (28) is positioned in this setting-rod receiving area (24).

2. The bone plate according to claim 1, **characterized in that** the bone-screw receiving opening (28) is positioned symmetrically in the setting-rod receiving area (24).

3. The bone plate according to one of the preceding claims, **characterized in that** the bone-screw receiving opening (28) is structured as a recessed receptacle for a countersunk screw head of the bone screw.

4. The bone plate according to one of the preceding claims, **characterized in that** the receiving opening (28) is spherical-shaped.

## Revendications

1. Lame plaque (10) pour l'ostéosynthèse comportant un corps de base (12) en forme de plaque, moyennant quoi le corps de base (12) présente une seule ouverture de réception (28) permettant de recevoir une vis à os fixant le corps de base (12) par exemple selon un certain angle, en ce que la tête de vis de la vis à os est reçue par l'ouverture de réception (28), moyennant quoi le corps de base (12) est doté d'une zone de réception (24) pour une barre de fixation, moyennant quoi la fixation de la barre de fixation sur le corps de base (12) est réalisée dans la zone de réception (24) par l'intermédiaire d'un couvercle (14), **caractérisée en ce que** le couvercle (14) est fixé de manière pivotante sur le corps de base (12) par l'intermédiaire d'une charnière (16), et **en ce que** l'ouverture de réception (28) est prévue à l'intérieur de cette zone de réception (24).

2. Lame plaque selon la revendication 1, **caractérisée en ce que** l'ouverture de réception (28) est prévue de manière symétrique à l'intérieur de la zone de réception (24).

3. Lame plaque selon l'une quelconque des revendications précédentes, **caractérisée en ce que** l'ouverture de réception (28) est conçue sous la forme d'un logement fraisé pour une tête de vis configurée sous la forme de tête fraisée de la vis à os.

4. Lame plaque selon l'une quelconque des revendications précédentes, **caractérisée en ce que** l'ouverture de réception (28) est configurée sous la forme d'une calotte.
